Europäisches Patentamt

European Patent Office

Office européen des brevets

(19)

(11) Publication number: **0 402 088**
**A2**

# EUROPEAN PATENT APPLICATION

(12)

(21) Application number: **90306082.0**

(51) Int. Cl.5: **A61K 39/21, A61K 39/095**

(22) Date of filing: **05.06.90**

(30) Priority: **06.06.89 US 362179**
**06.06.89 US 362178**
**06.06.89 US 362177**
**06.06.89 US 362176**

(43) Date of publication of application:
**12.12.90 Bulletin 90/50**

(84) Designated Contracting States:
**AT BE CH DE DK ES FR GB GR IT LI LU NL SE**

(71) Applicant: **MERCK & CO. INC.**
**126, East Lincoln Avenue P.O. Box 2000**
**Rahway New Jersey 07065-0900(US)**

(72) Inventor: **Emini, Emilio A.**
**6 Faggs Manor Lane**
**Paoli, PA 19301(US)**
Inventor: **Marburg, Stephen**
**50 Concord Avenue**
**Metuchen, New Jersey 08840(US)**
Inventor: **Scolnick, Edward M.**
**811 Wickfield Park Drive**
**Wynnewood, PA 19096(US)**
Inventor: **Larson, Vivian M.**
**362 Park Drive**
**Harleyville PA 19438(US)**

(74) Representative: **Hesketh, Alan, Dr. et al**
**European Patent Department Merck & Co.,**
**Inc. Terlings Park Eastwick Road**
**Harlow Essex, CM20 2QR(GB)**

(54) **Conjugate immunogen for aids.**

(57) A conjugate of the major neutralizing determinant of HIV, covalently linked to Neisseria outer membrane proteosome (Omp), is prepared and found to neutralize HIV after inoculation in monkeys. The conjugate is useful as a vaccine against AIDS or ARC as well as in the treatment of AIDS or ARC.

EP 0 402 088 A2

# CONJUGATE IMMUNOGEN FOR AIDS

Acquired Immune Deficiency Syndrome (AIDS) is the clinical manifestation of the apparent infection of CD4 helper T-cells and other cell targets by human immunodeficiency virus (HIV), also previously referred to as human T-lymphotropic virus type III (HTLV-III), Lymphoadenopathy-associated virus (LAV), or AIDS-related virus (ARV) (hereinafter collectively "HIV"). AIDS is a transmissible deficiency of cellular immunity characterized by opportunistic infections and certain malignancies. A similar disease. AIDS-related complex (ARC), shares many of the epidemiological features and immune abnormalities with AIDS, and often precedes the clinical manifestations of AIDS.

A vaccine against AIDS and/or ARC is an ideal prophylactic treatment for preventing the delibilitating effects of infection by HIV. Applicants have discovered an immunogen useful for such a vaccine. The immunogen is a covalent conjugate of the outer membrane proteosome (Omp) and one or more HIV envelope fragments.

Many of the details of the genetic function and virion structure of HIV have not yet been elucidated. However, certain general features have emerged. An RNA virus with a genome totaling about 9 kilobases (kb), its nucleotide sequence contains seven major open reading frames (ORFs) corresponding to the gag, pol and env, vif, tat, rev, and nef genes. The genes gag, pol and env code respectively for core subunits, viral enzymes such as reverse transcriptase or protease, and outer surface subunits. The gene vif codes for a viral infectivity factor, which is a protein involved with enhancement of cell-to-cell transmission of virions without affecting the budding process. The gene tat codes for a small protein required for viral infectivity, but its mechanism is not clear. The gene rev regulates expression of the viral proteins of gag, pol and env genes, possibly by facilitating transport of incompletely spliced RNA. The nef gene codes for a viral protein found in the cell cytoplasm, and it may modulate the host cellular signaling system and serve as a transciptional silencer. Terminal repeats in the nucleotide sequence are common to many retroviruses such as HIV and are required for viral replication and integration into the host chromosome. More recent discussions on the general nature of HIV genomic structure, replication and regulation are found in Ratner, L. et al. "Human T-Lymphotropic Retroviruses," in O'Brien, S.J. (ed.) Genetic Maps 1987 Cold Spring Harbor 1987 pp. 124-129; Franchini, G. et al., Nature 328, 539 (1987); Varmus, H. Genes & Dev 2, 1055 (1988).

Attempts to develop a vaccine to prevent infection with HIV generally have concentrated on the elicitation of specific virus-neutralizing antibodies. A region of the HIV surface coat protein (gp120) which is involved in the generation of such antibodies has been defined [Goudsmit et al., Proc. Natl. Acad. Sci USA 85, 4478 (1988); Ho et al., J. Virol. 61, 2024 (1987); Matsushita et al., J. Virol. 62, 2107 (1988); Palker et al., Proc. Natl. Acad. Sci. USA 85, 1932 (1988); Rusche et al., Proc. Natl. Acad. Sci. USA 85, 3198 (1988); Skinner et al., J. Virol. 62, 4195 (1988)]. However, attempts to use the intact viral coat protein or portions thereof to readily elicit significant levels of neutralizing antibodies have proven unsuccessful [Berman et al., Proc. Natl. Acad. Sci. USA 85, 5200 (1988); Hu et al., Nature 328, 721 (1987); Lasky et al.. Science 233, 209 (1986); Putney et al., Science 234, 1392 (1986); Robey et al., Proc. Natl. Acad. Sci. USA 83, 7023 (1986); Rusche et al., Proc. Natl. Acad. Sci. USA 84, 6924 (1987)].

Applicants are discoverers of a conjugate as a useful immunogen for AIDS. The conjugate elicits a substantial response of specific HIV-neutralizing antibodies in vivo. The conjugate consists essentially of a major neutralizing determinant of HIV covalently linked to purified outer membrane proteosome of Neisseria.

AIDS is a disease of a virus with a unique collection of attributes. HIV itself targets the immune system; it possesses a reverse transcriptase capable of turning out highly mutated progeny: it is sequestered from the immune system and it has a hypervariable surface in the (env) region. See, e.g. Hilleman, M.R., Vaccine 6, 175 (1988); Barnes, D.M., Science 240, 719 (1988). In view of these attributes, it was neither anticipated nor expected that the conjugates of this invention would serve as AIDS vaccines.

BRIEF DESCRIPTION OF THE INVENTION

Synthetic peptide(s), representing a major neutralization determinant (MNtD) of the human immunodeficiency virus (HIV), are chemically synthesized and conjugated with purified Neisseria meningitidis outer membrane proteosome (Omp). The resulting MNtD-omp conjugate, when inoculated into test animals, readily elicited high levels of HIV-neutralizing antibodies. The conjugate is useful as a practical immunogen for eliciting a biologically important immune response against HIV.

ABBREVIATIONS AND DEFINITIONS

AIDS
Acquired immune deficiency syndrome
ARC
AIDS-related complex
conjugation
The process of covalently attaching 2 molecules each containing one or more immunological determinants, e.g., HIV envelope fragments and Omp
conjugate
Result of conjugation, also known as an antigenic conjugate or immunological conjugate
HIV
Generic term for the presumed etiological agent of AIDS and/or ARC, also referred to as strains HTLV-III, LAV, and ARV.
MNtD
Major neutralization determinant of HIV
Omp
Outer membrane proteosome
Recombinant protein
A polypeptide or oligopeptide expressed by foreign DNA in a recombinant eukaryotic or procaryotic expression system.
Recombinant expression system
A cell containing a foreign DNA expressing a foreign protein or a foreign oligopeptide.

| Amino Acids | | |
|---|---|---|
| Full Name | Three-letter symbol | One-letter symbol |
| Alanine | Ala | A |
| Arginine | Arg | R |
| Asparagine | Asn | N |
| Aspartic acid | Asp | D |
| Asn and/or Asp | Asx | B |
| Cysteine | Cys | C |
| Glutamine | Gln | Q |
| Glutamic acid | Glu | E |
| Gln and/or Glu | Glx | Z |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |

The terms "protein," "peptide," "oligopeptide," and "polypeptide" and their plurals have been used interchangeably to refer to chemical compounds having amino acid sequences of five or more amino acids. "Amino acid" refers to any of the 20 common amino acids for which codons are naturally available, and are listed in the table of amino acids given above.

When any variable (e.g. $J_1$, $J_2$, C, G, etc.) occurs more than one time in any constituent or in Formula II, its definition on each occurrence is independent of its definition at every other occurrence. Also, combinations of substituents and/or variables are permissible only if such combinations result in stable

3

compounds.

## DETAILED DESCRIPTION OF THE INVENTION

The present invention provides an effective vaccine against AIDS or ARC, and comprises an antigenic conjugate of the formula

$(MNtD)_n \sim (Omp)$   I,

wherein:

MNtD is the major neutralization determinant of HIV, which is a polypeptide of one or more amino acid sequences;

n = 1-50, wherein n is the number of polypeptides of MNtD covalently linked to Omp;

$\sim$ indicates covalent linkage;

Omp is outer membrane proteosome of the microorganism Neisseria.

The conjugate is provided for immunological purposes. It serves as a novel and useful antigen in the treatment or prevention of AIDS or ARC. It is the prinicipal constituent of an AIDS vaccine, to be used pre- or post-exposure to prevent or treat HIV infection or disease.

An example of a formula I conjugate is $(NNTRKSIRIQRGPGRAFVTIGKIGN)_{3.3}$-Omp, which is Omp covalently linked to an HIV peptide fragment (IIIB isolate, see below) with an average of 3.3 HIV peptide fragments for each Omp. The number 3.3 indicates the degree of substitution or the number of polypeptides.

Other examples readily occur to one of ordinary skill in the art of immunology. See also the illustrations of Example 3B.

## HIV Major Neutralization Determinant

The immunological determinant conferring specificity to the HIV neutralizing antibodies in this invention is the major neutralization determinant (abbreviated MNtD). The major neutralization determinant of this invention is defined in the immunological arts as any peptide determinant immunologically cross-reactive with one or more of those peptides having sequences of the following Table:

## Table I

YNKRKRIHIGPGRAFYTTKNIIGTI or the MN ioslate,
NNTTRSIHIGPGRAFYATGDIIGDI of the SC isolate, or
NNTRKSIRIQRGPGRAFVTIGKIGN of the IIIB isolate.

Immunological cross-reactivity can be measured by any conventional method, e.g., virus-neutralization, agglutination, radioimmunoassay, enyme-linked immunoassay, or cell-mediated immunity.

The IIIB protein sequence given above has a narrower spectrum of cross-reactive determinants than the MN or SC protein sequence. Accordingly it is also advantageously used as part of a cocktail of antigenic conjugates comprising conjugates of Omp covalently bound to MNtD peptides of more than a single sequence. It is ordinary and conventional practice in the immunological arts to determine the appropriate cocktail of antigenic conjugates by evaluating neutralizing antibody response characteristics, including determination of titer and cross-reactivity with polypeptides selected by the investigators.

Alternatively, the MNtD can be defined in another embodiment as any peptide having a sequence of 44 amino acids or less, but at least 5 amino acids in length, of the formula;

$C-J_1-G-X-G-J_2-C$   II

wherein:

C is absent or cysteine;

$J_1$ is absent or any peptide up to 19 amino acids in length;

G is glycine;

X is proline, leucine, alanine, glutamine, or serine; and

$J_2$ is absent or any peptide of up to 20 amino acids in length;

or pharmaceutically acceptable salt thereof. Like all proteins or peptide sequences in their standard formats,

the sequence of formula II reads with the amino terminus on the "left" side and the COOH terminus on the "right" side.

One preferred embodiment of formula II is where X is proline.

Another preferred embodiment of formula II provides that is proline, C is present twice, $J_1$ and $J_2$ are present.

Another preferred embodiment of formula II is any of the three amino acid sequences of Table I.

For example, MNtD according to Formula II may be any of the following known and isolated sequences, as provided in the following Table II.

## Table II
### Sample of Isolated Equivalents of MNtD

| Official Designation | Amino Acid Sequence |
|---|---|
| MN | CTRPNYNK RK R IHIGPGR AFYTTKNIIGTIRQAHC |
| SC | -----N-T TR S ------- ---A-GD---D------ |
| WMJ3 | D----DIA -R - ------- ---- GE-R-N------ |
| WMJ1 | -----N-V -R H ------- ---- GE-R-N------ |
| WMJ1.5 | -----N-V -R -H ------- --- YGE-R-N------ |
| CC | ----- -N T- KG ------- -V--ARR---D------ |
| CHILDES | -----N-T -- S ------- -I-A-AR---D------ |
| WMJ3.3 | -----DIA - -R ------- ---YG- ----N------ |
| J.GOUD 1 | -----N-T -- S ------- ---A-QK---K------ |
| 6587-4 | ----YS-V -N - ------- --H---R-T-DM---R- |
| WMJD | ---- - NV- R-H ------- ---- GE-R-N------ |
| CHILDES | --- - NT--S ------- -I-A-AR---D------ |
| J.GOUD 2 | -----N-T -- S -N----- -G-A-GQ---N------ |
| BAL | -----N-T -- S -S---- -----GE---D------ |
| SF2 | -----N-T -- S -Y----- --H--GR---D--K--- |
| SF4 | -----N-T -- S -Y----- --H--GR---D--K--- |
| RJS4.26 | ---- ---K -I -H M----- ---A-GG-M-D------ |
| NY5 | -----N-TK - G -A---- TL-AREK---D------ |
| 2321 | -M---N-T -- S -S---- ---FA-GD---D------ |
| JWK264 | -----N-T - -S ------WSVH---GE-V-D------ |
| 6587-7 | N- YT-- G------- -I-AIGD---D------ |
| IIIB(BH10) | ------ -NT --SIR -QR---- ---V-IGK- -NM------ |
| 6587-3 | --N-TRA-LS - ---- S---A-R---V- |
| WMJ-2 | ---- --N VR -S LS---- ---R- RE---I------ |
| WMJ2.3 | ---- --N VR -S LS---- -- RYRE---I------ |
| RF | -----N-T -- S -TK---- VI-A-GQ---D--K--- |
| CDC42 | ---- -NT -- - VTL---- VW---GE-L-N------ |
| CDC451 | ----- -HT -- - VTL---- VW---GE-L-N------ |

| Official Designation | Amino Acid Sequence |
|---|---|
| Z3 | ----GSD-KI-QS   -R----- KV--- A-GG-- - G----- |
| RUTZ | Y-NI---GTHVG-V----- -I-A-N---K-SGN |
| LAV-MAL | ---- G- NT-  -G --F---- Q-L---- G-V-D---R-Y- |
| SF33 | ------ -N  -R -R -TS---- KVL----GE----D---K-Y- |
| SF170 | ------N-T  ---  S GT---- Q----A-GD----D----Y- |
| LAVELI | -A--- - QNT- Q--  TP---L- QSL--- RSRSI-G----- |
| Z6 | ---- - -NT-QSTP -  -L- Q-L---RGRTKI-G----- |
| JY1 | ---- D---IT-QSTP -  -L- Q-L---- T-K-D-----Y- |

NOTE: Dashes in Table II indicate that the substitution is the same as the defining letter of the first line. A letter is a substitution only for that line. Thus, for example, the first three amino acid sequences of the first four isolates are, respectively, CTR, CTR, DTR, CTR.

It will be understood that the novel products of the present invention encompass a range HIV peptide sequences, prepared either by recombinant expression systems or synthetic methods or both. For example, variations by conservative substitution in the HIV amino acid or peptide sequence [defined as sets in Table 1 of Taylor, W.R., J. Mol. Biol. 188, 233 (1986)] generally will not result in any substantial or novel modification of the principles and practice of the present invention. Alternatively, deletion(s) within the formula II peptide regions are encompassed by the present vaccines. Still other variations may occur in recombinant expression systems and include, by way of example, post-translational modifications, proteolytic processing, adenylation, carboxylation, glycosylation, hydroxylation, methylation, phosphorylation or myristoylation. It will be understood that MNtD or fragment or variant thereof in this application includes any such variations or portions of the amino acid sequence, whether by conservative amino acid substitution, deletion, post-translational processing, ribosome frame shifting or other process, provided that the resulting MNtD, fragment or variant thereof, is immunologically cross-reactive with at least one of the peptides listed in Table I above. Furthermore, it is stipulated that MNtD, fragment or variant thereof is 5 or more amino acids in length.

By way of illustration, one embodiment of the present invention is a covalent conjugate of Omp and the determinant having the peptide sequence of
YNKRKRIHIGPGRAFYTTKNIIGTI (of the MN isolate).

Another embodiment of the present invention is a covalent conjugate of Omp and the determinant having the peptide sequence of
NNTTRSIHIGPGRAFYATGDIIGDI (of the SC isolate).

Another embodiment of the present invention is a covalent conjugate of Omp and the determinant having the peptide sequence of
NNTRKSIRIQRGPGRAFVTIGKIGN (of the IIIB isolate).

A further embodiment of the present invention is a covalent conjugate of Omp and the determinant having the consensus peptide sequence
LNESVEINCTRPNNNTRKSIHIGPGRAFYTTGRIIGDIRQAHC.
This consensus sequence contains the preferred amino acid at each position of the composite provided in Table II.

A further embodiment of the present invention is a covalent conjugate of Omp and the determinant having the peptide sequence
NNTRKSIHIGPGRAFYTTGRIIGDI, which is a fragment of the consensus sequence of the preceeding paragraph.

Other embodiments of the present invention are covalent conjugates of Omp and any fragment of the consensus sequence, provided the fragment is 5 or more amino acids in length.

Preparation of Major Neutralization Determinant

## A. Organic Synthesis of MNtD:

Standard and conventional methods exist for rapid and accurate synthesis of long peptides on solid-phase supports. Solution-phase synthesis is usually feasible only for selected smaller peptides.

Synthesis on solid-phase supports, or solid-phase synthesis, is most conveniently performed on an automated peptide synthesizer according to e.g., Kent, S. et al., "Modern Methods for the Chemical Synthesis of Biologically Active Peptides," in Alitalo, K. et al., (eds.). Synthetic Peptides in Biology and Medicine, Elsevier 1985, pp. 29-57. Manual solid-phase synthesis may be employed instead, by following the classical Merrifield techniques, as described, for example, in Merrifield, R.B. J. Am. Chem. Soc. 85, 2149 (1963), or known improvements thereof. Solid-phase peptide synthesis may also be performed by the Fmoc method, which employs very dilute base to remove the Fmoc protecting group. Segment synthesis-condensation is a further variant of organic synthesis of peptides as within the scope of the techniques of the present invention.

In organic synthesis of peptides, protected amino acids are condensed to form amide or peptide bonds with the N-terminus of a growing peptide. Condensation is usually performed with the carbodiimide method by reagents such as dicyclohexylcarbodiimide, or N-ethyl, $N_1$-(γ-dimethylaminopropyl) carbodiimide. Other methods of forming the amide or peptide bond include, but are not limited to, synthetic routes via an acid chloride, azide, mixed anhydride or activated ester. Common solid-phase supports include polystyrene or polyamide resins.

The selection of protecting groups of amino acid side claims is, in part, dictated by particular coupling conditions, in part by the amino acid and peptide components involved in the reaction. Such amino-protecting groups ordinarily employed include those which are well known in the art, for example, urethane protecting substituents such as benzyloxycarbonyl (carbobenzoxy), p-methoxycarbobenzoxy, p-nitrocarbobenzoxy, t-butyloxycarbonyl, and the like. It is preferred to utilize t-butoxycarbonyl (BOC) for protecting the ε-amino group, in part because the BOC protecting group is readily removed by relatively mild acids such as trifluoroacetic acid (TFA), or hydrogen chloride in ethyl acetate.

The OH group of Thr and Ser may be protected by the Bzl (benzyl) group and the ε-amino group of Lys may be protected by the isopropoxycarbonyl (IPOC) group or the 2-chlorobenzyloxycarbonyl (2-Cl-CBZ) group. Treatment with HF or catalytic hydrogenation are typically employed for removal of IPOC or 2-Cl-CBZ.

For preparing cocktails of closely related peptides, see, e.g., Houghton, R.A., Proc. Natl. Acad. Sci. USA 82, 5131 (1985).

## B. Expression of MNtD in a Recombinant Expression System

It is now a relatively straightforward technology to prepare cells expressing a foreign gene. Such cells act as hosts and include E. coli, B. subtilis, yeasts, fungi, plant cells or animal cells. Expression vectors for many of these host cells have been isolated and characterized, and are used as starting materials in the construction, through conventional recombinant DNA techniques, of vectors having a foreign DNA insert of interest. Any DNA is foreign if it does not naturally derive from the host cells used to express the DNA insert. The foreign DNA insert may be expressed on extrachromosomal plasmids or after integration in whole or in part in the host cell chromosome(s), or may actually exist in the host cell as a combination of more than one molecular form. The choice of host cell and expression vector for the expression of a desired foreign DNA largely depends on availability of the host cell and how fastidious it is, whether the host cell will support the replication of the expression vector, and other factors readily appreciated by those of ordinary sHill in the art.

The technology for recombinant procaryotic expression systems is now old and conventional. The typical host cell is E. coli. The technology is illustrated by treatises such as Wu, R (ed) Meth. Enzymol. 68 - (1979) and Maniatis, T. et. al., Molecular Cloning: A Laboratory Manual Cold Spring Harbor 1982.

The foreign DNA insert of interest comprises any DNA sequence coding for MNtD fragment or variant thereof, including any synthetic sequence with this coding capacity or any such cloned sequence or combination thereof. For example, MNtD peptide coded and expressed by an entirely recombinant DNA sequence is encompassed by this invention.

Vectors useful for constructing eukaryotic expression systems for the production of recombinant MNtD comprise the DNA sequence for MNtD, fragment or variant thereof, operatively linked thereto with appropriate transcriptional activation DNA sequences, such as a promoter and/or operator. Other typical features may include appropriate ribosome binding sites, termination codons, enhancers, terminators, or replicon elements. These additional features can be inserted into the vector at the appropriate site or sites by conventional splicing techniques such as restriction endonuclease digestion and ligation.

Yeast expression systems, which are one variety of recombinant eukaryotic expression systems, generally employ Saccharomyces cerevisiae as the species of choice for expressing recombinant proteins. S. cerevisiae and similar yeasts possess well known promoters useful in the construction of yeast expression systems, including but not limited to GAP491, GAL10, ADH2, and alpha mating factor.

Yeast vectors useful for constructing recombinant yeast expression systems for expressing MNtD include, but are not limited to, shuttle vectors, cosmid plasmids, chimeric plasmids, and those having sequences derived from 2-micron circle plasmids.

Insertion of the appropriate DNA sequence coding for MNtD, fragment or variant thereof, into these vectors will, in principle, result in a useful recombinant yeast expression system for MNtD where the modified vector is inserted into the appropriate host cell, by transformation or other means.

Recombinant mammalian expression systems are another means of producing the recombinant MNtD for the conjugates of this invention. In general, a host mammalian cell can be any cell that has been efficiently cloned in cell culture. Host mammalian cells useful for the purposes of constructing a recombinant mammalian expression system include, but are not limited to, Vero cells, NIH3T3, GH3, COS, murine C127 or mouse L cells. Mammalian expression vectors can be based on virus vectors, plasmid vectors which may have SV40, BPV or other viral replicons, or vectors without a replicon for animal cells. Detailed discussions on mammalian expression vectors can be found in the treatises of Glover, D.M. (ed.) "DNA Cloning: A Practical Approach," IRL 1985, Vols. I and II.

Recombinant MNtD may possess additional and desirable structural modifications not shared with the same organically synthesized peptide, such as adenylation, carboxylation, glycosylation, hydroxylation, methylation, phosphorylation or myristoylation. These added features may be chosen or preferred as the case may be, by the appropriate choice of recombinant expression system. On the other hand, recombinant MNtD may have its sequence extended by the principles and practice of organic synthesis of section A above.


Outer Membrane Proteosome, Its Variants, Purification


The immunological enhancer of the present invention is purified outer membrane proteosome (Omp) of Neisseria.

Outer membrane proteosome may be prepared from any species of the gram-negative cocci known as Neisseria, including but not limited to N. meningitidis, N. lactamica. N. sicca. N. flavescens. N. subflava, and N. mucosa. Preferred preparations are derived from N. meningitidis, e.g., of groups A, B or C. Most preferred is Omp of N. meningitidis group B.

Various methods of purifying Omp are known, any of which are suitable for preparing useful quantities of Omp for conjugation to MNtD. C. Frasch has described washing N. meningitidis cells in salt solutions, followed by treatment with detergent such as deoxycholate. See, for example, C.E. Frasch et al., J. Exp. Med. 140, 87 (1974): C.E. Frasch J. Bact. 127, 973 (1976); and C.E. Frasch et al., J. Exp. Med. 147, 629 (1978). T.B. Helting has formulated another known method which employs direct detergent extraction of N. meningitidis cells without salt. See, for example, T.B. Helting et al., Acta Path. Microbiol. Scand. Sect. C. 89, 69 (1981) and U.S. Patent 4,271,147.


Conjugation of MNtD and Omp to Form a Covalent Linkage(s) Yielding Conjugate


Antigenic conjugates of MNtD and Omp are useful for vaccination against AIDS or ARC. Such conjugates have at least one covalent linkage between the antigen MNtD and Omp, and typically have more than one MNtD convalently bound to each Omp.

MNtD and Omp are prepared separately, then linked by non-specific cross-linking agents, monogeneric spaces or bigeneric spacers. Methods for non-specific cross-linking include, but are not limited to, reaction with glutaraldehyde; reaction with N-ethyl-N'-(3-dimethylaminopropyl) carbodiimide, with or without admix-

ture of a succinylated carrier; periodate oxidation of glycosylated substituents followed by coupling to free amino groups of a protein carrier in the presence of sodium borohydride or sodium cyanoborohydride; diazotization of aromatic amino groups followed by coupling on tyrosine side chain residues of the protein; reaction with isocyanates: or reaction of mixed anhydrides. See, generally, Briand, J. P. et al. J. Imm. Meth. 78, 59 (1985). These methods of non-specifically cross-linking are conventional and well-known in the typical practice of preparing conjugates for immunological purposes.

In another embodiment of the invention conjugates formed with a monogeneric spacer are prepared. These spacers are bifunctional and require functionalization of only one of the partners of the reaction pair to be conjugated before conjugation takes place.

By way of illustration rather than limitation, an example of a monogeneric spacer involves coupling the polypeptide MNtD to one end of the bifunctional molecule adipic acid dihydrazide in the presence of carbodiimide. A diacylated hydrazine presumably forms with pendant glutamic or aspartic carboxyl groups of MNtD. Conjugation then is performed by a second coupling reaction with Omp in the presence of carbodiimide. For similar procedures, see for example, Schneerson, R. et al., J. Exp. Med. 152, 361 (1980). Another example of a monogeneric spacer is described in Fujii, N. et al. Int. J. Peptide Protein Res. 26, 121 (1985).

In a preferred embodiment of the invention conjugates of Omp and MNtD are formed with a bigeneric spacer as in Example 3. These spacers are formed after each partner of the reaction pair to be conjugated, e.g., Omp and MNtD, is functionalized with a bifunctional spacer. Conjugation occurs when each functionalized partner is reacted with its opposite partner to form a stable covalent bond or bonds. See, for example, Marburg, S. et al., J. Am. Chem. Soc. 108, 5282-5287 (1986) and Marburg, S. et al., U.S. Patent 4,695,624, issued 22 September 1987, each incorporated by reference. Bigeneric spacers are preferred for preparing conjugates in human vaccines since the conjugation reaction is well characterized and easily controlled.

Typical and conventional immunological practice provides for the ready and easy synthesis of antigenic conjugates within the scope of the present invention, including the conjugation of Omp with virtually any desired degree of substitution of virtually any peptide. Heterogeneous products of the conjugation reaction are easily separable if needed by a variety of suitable column chromatography techniques.


Vaccine Formulation


The form of the immunogen within the vaccine takes various molecular configurations. A single molecular species of the antigenic conjugate $(MNtD)_n$-(Omp) will often suffice as a useful and suitable antigen for the prevention or treatment of AIDS or ARC. Other antigens in the form of cocktails are also advantageous, and consist of a mixture of conjugates that differ by, for example, the degree of substitution (n) or the amino acid sequence of MNtD or both.

An immunological vector, carrier or adjuvant may be added as an immunological vehicle according to conventional immunological testing or practice.

Adjuvants may or may not be added during the preparation of the vaccines of this invention. Alum is the typical and preferred adjuvant in human vaccines, especially in the form of a thixotropic, viscous, and homogeneous aluminum hydroxide gel. For example, one embodiment of the present invention is the prophylactic vaccination of patients with a suspension of alum adjuvant as vehicle and a cocktail of $(MNtD)_n$-OMP as the selected set of immunogens or antigens.

The vaccines of this invention may be effectively administered, whether at periods of pre-exposure and/or post-exposure, in combination with effective amounts of the AIDS antivirals, immunomodulators, antibiotics, or vaccines of Table III [source: Market Letter, Nov. 30, 1987, p. 26-27: Genetic Engineering News, Jan. 1988, Vol. 8, p. 23.]

## TABLE III[1]

### A. Antivirals

| Drug Name | Manufacturer | Indication |
|---|---|---|
| AL-721 | Ethigen | ARC, PGL |
| BETASERON (interferon beta) | Triton Biosciences | AIDS, ARC, KS |
| CARRISYN (polymannoacetate) | Carrington Labs | ARC |
| CYTOVENE (ganciclovir) | Syntex | CMV |
| DDC (dideoxycytidine) | Hoffmann-La Roche | AIDS, ARC |
| FOSCARNET (trisodium phosphonoformate) | Astra AB | HIV inf, CMV retinitis |
| HPA-23 | Rhone-Poulenc Sante | HIV infection |
| ORNIDYL (eflornithine) | Merrell Dow | PCP |

---

[1]Abbreviations: AIDS (Acquired Immune Deficiency Syndrome); ARC (AIDS related complex); CMV (Cytomegalovirus, which causes an opportunistic infection resulting in blindness or death in AIDS patients); HIV (Human Immunodeficiency Virus, previously known as LAV, HTLV-III or ARV); KS (Kaposi's sarcoma); PCP (Pneumonocystis carinii pneumonia, an opportunistic infection); PGL (persistent generalized lymphadenopathy).

| Drug Name | Manufacturer | Indication |
|---|---|---|
| PEPTIDE T (octapeptide sequence) | Peninsula Labs | AIDS |
| RETICULOSE (nucleophospho-protein) | Advanced Viral Research | AIDS, ARC |
| IR (zidovudine; AZT) | Burroughs Wellcome | AIDS, advanced ARC<br><br>pediatric AIDS, KS, asympt HIV, less severe HIV, neurological involvement. |
| RIFABUTIN (ansamycin LM 427) | Adria Labs | ARC |
| (trimetrexate) | Warner-Lambert | PCP |
| UA001 | Ueno Fine Chem Industry | AIDS, ARC |
| VIRAZOLE (ribavirin) | Viratek/ICN | AIDS, ARC, KS |
| WELLFERON (alfa interferon) | Burroughs Wellcome | KS, HIV, in comb with RETROVIR |
| ZOVIRAX (acyclovir) | Burroughs Wellcome | AIDS, ARC, in comb with RETROVIR |

## B. Immunomodulators

| Drug Name | Manufacturer | Indication |
|---|---|---|
| ABPP (bropirimine) | Upjohn | Advanced AIDS, KS |
| AMPLIGEN (mismatched RNA) | DuPont HEM Research | ARC, PGL |
| (Anti-human alpha interferon antibody) | Advanced Biotherapy Concepts | AIDS, ARC, KS |
| Colony Stimulating Factor (GM-CSF) | Sandoz Genetics Institute | AIDS, ARC, HIV, KS |
| CL246,738 (CL246,738) | American Cynamid | AIDS |
| IMREG-1 | Imreg | AIDS, ARC, PGL, KS |
| IMREG-2 | Imreg | AIDS, ARC, PGL, KS |
| IMUTHIOL (diethyl dithio carbamate) | Merieux Institute | AIDS, ARC |
| IL-2 (interleukin-2) | Cetus | AIDS, KS |

| Drug Name | Manufacturer | Indication |
|---|---|---|
| IL-2<br>(interleukin-2) | Hoffmann-La Roche<br>Immunex | AIDS, KS |
| INTRON-A<br>(interferon alfa) | Schering-Plough | KS |
| ISOPRINOSINE<br>(inosine pranobex) | Newport<br>Pharmaceuticals | ARC, PGL, HIV<br>seropositive<br>patients |
| (methionine<br>enkephalin) | TNI<br>Pharmaceuticals | AIDS, ARC |
| MTP-PE<br>(muramyl-tripep-<br>tide) | Ciba-Geigy | KS |
| THYMOPENTIN (TP-5)<br>(thymic compound) | Ortho<br>Pharmaceuticals | HIV infection |
| ROFERON<br>(interferon alfa) | Hoffmann-La Roche | KS |
| (recombinant<br>erythropoietin) | Ortho<br>Pharmaceuticals | severe anemia<br>assoc with AIDS<br>& RETROVIR<br>therapy |
| TREXAN<br>(naltrexone) | DuPont | AIDS, ARC |
| TNF (tumor<br>necrosis factor) | Genentech | ARC, in combination<br>interferon gamma |

## C. Antibiotics

PENTAM 300          LyphoMed          PCP

(pentamidine

isethionate)

## D. Vaccines

Gag                    Merck          AIDS,ARC

It will be understood that the scope of combinations of the vaccines of this invention with AIDS antivirals, immunomodulators, antibiotics or vaccines is not limited to the list in the above Table, but includes in principle any combination with any pharmaceutical composition useful for the treatment of AIDS. The AIDS or HIV vaccines of this invention include vaccines to be used pre- or post-exposure to prevent or treat HIV infection or disease, and are capable of producing an immune response specific for the immunogen.

EXAMPLE 1

Preparation of Synthetic Peptides

A. The oligopeptide NNTRKSIRIQRGPGRAFVTIGKIGN, also known as RP135, was synthesized by conventional solid-phase techniques on an automated peptide synthesizer, according to Kent, S. et al., "Modern Methods for the Chemical Synthesis of Biologically Active Peptides," in Alitalo, K. et al.(eds.), Synthetic Peptides in Biology and Medicine, Elsevier 1985, pp. 29-57. The peptide was secured to a methylbenzhydrylamine support through the beta COOH-terminus of aspartic acid. Cleavage with HF as a final step resulted in COOH-terminal asparagine.

B. Other peptides are also synthesized, as provided in the following Table:

Table

| Sequence | Derived from Isolate |
|---|---|
| NNTRKSITKGPGRVIYATGQIIGDIN | RF |
| YNKRKRIHIGPGRAFYTTKNIIGTIN | MN |
| KNNTTRSIHIGPGRAFYATGDIIGDIN | SC |
| KNNVRRSLSIGPGRAFRTREIIGIIRN | WMJ2 |
| NNTRKSIHIGPGRAFYTTGRIIGDIN | Variant 1 |
| NNTRKSIHIGLGRAFYTTGRIIGDIN | Variant 2 |
| NNTRKSIHIGAGRAFYTTGRIIGDIN | Variant 3 |
| NNTRKSIHIGSGRAFYTTGRIIGDIN | Variant 4 |
| NNTRKSIHIGQGRAFYTTGRIIGDIN. | Variant 5 |

EXAMPLE 2

Extraction and Purification of Omp

A. First Method

All materials. reagents and equipment were sterilized by filtration, steam autoclave or ethylane oxide, as appropriate: asceptic technique was used throughout.

A 300 gm (wet weight) aliquot of 0.5% phenol inactivated cell paste of Meningococcal group B11 was suspended in 1200 mls of distilled water than suspended by stirring magnetically for 20 minutes at room temperature. The suspended cells were pelleted at 20,000 xg for 45 minutes at 5°C.

For extraction, the washed cells were suspended in 1500 mls 0.1 M Tris, 0.01 M EDTA Buffer pH 8.5 with 0.5% sodium deoxychloate (TED Buffer) and homogenized with a 500 ml Sorvall omnimixer at setting 3 for 60 seconds. The resulting suspension was transferred to ten Erlenmeyer flasks (500 ml) for extraction in a shaking water bath for 15 minutes at 56°C. The extract was centrifuged at 20,000 x g for 90 minutes at 5°C and the viscous supernatant fluid was decanted (volume = 1500 mls). The decanted fluid was very turbid and was recentrifuged to clarify further at 20,000 x g for 90 minutes at 5°C. The twice spun supernatant fluid was stored at 5°C. The extracted cell pellets were resuspended in 1500 mls TED Buffer. The suspension was extracted for 15 minutes at 56°C and recentrifuged at 20,000 x g for 90 minutes. The supernatant fluids which contained purified Omp were decanted (volume = 1500 mls) and stored at 5°C.

B. Second Method

All material. reagents, equipment and filters were sterilized by heat, filtration or ethylene oxide. One exception was the K-2 ultracentrifuge which was sanitized with a 0.5% formal in solution. Laminar flow canopies provided sterility protection during equipment connections. Aseptic techniques were followed throughout the entire operations. Overnight storage of the protein was at 2-8°C between steps. A 0.2 micron sterile filtration was conducted just before the final diafiltration to ensure product sterility.

Two 600-liter batches of Neisseria meningitidis were fermented and killed with 0.5% phenol, then concentrated to roughly 25 liters using two 10 ft² 0.2 micron polypropylene cross-flow filtration membranes. The concentrated broth then was diafiltered with 125 liters of cell wash buffer (0.11 M Sodium Chloride, 17.6 mM Sodium Phosphate Diabasic. 23.3 mM Ammonium Chloride, 1.34 mM Potassium Chloride, adjusted to pH 7 with 85% Phorphoric Acid followed by 2.03 mM Magnesium Sulfate Heptahydrate).

For extraction, an equal volume of 2X-TED buffer (0.2M Tris. 0.02M EDTA adjusted to pH 8.5 with concentrated HCl followed with the addition of 1.0% sodium deoxycholate) was added to the cell slurry. The resulting slurry was heated to 56 ± 3°C and maintained at this temperature for 30 minutes to complete the extraction of Omp from the cells.

For further purification, the extracted cell slurry was centrifuged at 30,000 x g (18,000 rpm) in a "one-pass" flow mode in a K-ultracentrifuge, and the supernatant stream was collected. The low-speed supernatant was concentrated to 10 liters on two 0.1-micron polysulfone autoclavable hollow-fiber membranes and collected in an 18 liter sterile bottle. The filtration equipment was given two 4-liter rinses with TED buffer (0.1M Tris, 0.01M EDTA, adjusted to pH 8.5 with concentrated HCl, followed with the addition of sodium deoxycholate to 0.5%) which was combined with the retentate. The retentate was subdivided into two or three equal parts. Each part was centrifuged at 80,000 x g (35,000 rpm) for 30 mintues. The Omp protein was pelleted, and the majority of soluble proteins, nucleic acids and endotoxins remained in the supernatant. The supernatant was discarded. The pelleted protein was resuspended by recirculating 55 ± 5°C TED buffer through the rotor. The first high-speed resuspensions were combined and subjected to a second low-speed spin. The second low-speed spin ensured that residual cell debris was removed from the product stream. The second low speed supernatant was subdivided into two or three equal parts. Each fraction was given two consecutive high-speed spins. All high-speed spins were operated under the same conditions and each further purified the Omp protein.

For sterile filtration and final diafiltration. the third high-speed resuspensions were diluted with an equal volume of TED buffer and filtered through a 0.2 micron cellulose acetate filter. When all fractions were permeated, an 8 L TED buffer rinse was used to flush the filtration system. The permeate and rinse were combined and concentrated to 3 liters on a 0.1 micron polysulfone autoclavable hollow fiber membrane. The material then was diafiltered with 15 liters of sterile pyrogen free water. The retentate was collected in a 4-liter bottle along with a 1-L rinse to give the final product. The final aqueous suspension was stored at 2-8°C, as purified Omp.

C. Third Method

Omp is purified from 0.2 M LiCl-0.1M Na Acetate, pH 5.8, extracts by ultracentrifugation, by the method of C.E. Frasch et al. J. Exp. Med. 140, 87-104 (1974), herein incorporated by reference.

EXAMPLE 3

A. Preparation of RP135 peptide (NNTRKSIRIQRGPGRAFVTIGKIGN)-Omp conjugate ("RP135-Omp" conjugate)

N-acetylhomocystaminylated outer membrane protein (Omp) of N. meningitidis from 59 mg of Omp (purified by Method B of Example 2) was prepared by the centrifugation method described in Marburg, S. et al., J. Am. Chem. Soc. 108:5282 (1986). This material (about 50 mg) was reacted at pH 8 (6.5 mL 0.1M $\overline{PO_4}$ buffer) with 9.8 mg of N-$\Omega$-bromoacetylated RP135 (lyophilized) under $N_2$ for 18 hours at room temperature.

The reaction mixture was diluted to 10 mL with $H_2O$ and centrifuged for 2h, at 4°C and 43,000 rpm. The supernatant was removed, and the pellet resuspended, using a Dounce tissue homogenizer, in 10 mL of $H_2O$. This suspension was recentrifuged (as above) and the pellet resuspended in 9.5 mL of $H_2O$. A low speed spin for 1 minute in a clinical centrifuge removed a flocculent insoluble material. A Lowry protein assay of the supernatant affords a value of 370 $\mu$g protein/mL (about a 5% yield) and a Spinco amino acid analysis affords a S-carboxymethylhomo-cysteine/lysine ratio of .005. Computations of amino acid values indicate the degree of substitution is about 3.3 peptides per protein monomer. Yield: 3.7 mg of (RP135)$_{3.3}$-Omp conjugate, hereafter "RP135-Omp conjugate."

B. Preparation of Other Peptide Conjugates

By the method of Example 3A the following peptide-Omp conjugates are obtained.
(NNTRKSITKGPGRVIYATGQIIGDIN)$_5$-Omp,
(YNKRKRIHIGPGRAFYTTKNIIGTIN)$_4$-Omp,
(KNNTTRSIHIGPGRAFYATGDIIGDIN)$_6$-Omp,
(KNNVRRSLSIGPGRAFRTREIIGIIRN)$_8$-Omp,
(NNTRKSIHIGPGRAFYTTGRIIGDIN)$_{10}$-Omp,
(NNTRKSIHIGLGRAFYTTGRIIGDIN)$_{6.5}$-Omp,
(NNTRKSIHIGAGRAFYTTGRIIGDIN)$_{3.3}$-Omp,
(NNTRKSIHIGSGRAFYTTGRIIGDIN)$_{4.0}$-Omp,

EXAMPLE 4

Protocol for Inoculation of Animals with the RP135-Omp Conjugate

Alum was used as an adjuvant during the inoculation series. The inoculum was prepared by dissolving the RP135-Omp conjugate in physiologic saline at a final conjugate concentration of 100 $\mu$g/ml. Preformed alum (aluminum hydroxide gel) was added to the solution to a final level of 500 $\mu$g/ml aluminum. The conjugate was allowed to adsorb onto the alum gel for two hours at room temperature. Following adsorption, the gel with the conjugate was washed twice with physiologic saline and resuspended in the saline to a protein concentration of 100 μg/ml.

African green monkeys were individually inoculated with four 100 mcg doses of the RP135-omp conjugate adsorbed onto alum. Each dose was injected intramuscularly. The doses were delivered one or five months apart (week 0, 4, 8 and 28). The animals were bled at intervals of two or four weeks. Serum samples were prepared from each bleed to assay for the development of specific antibodies as described in the subsequent examples.

EXAMPLE 5

Analysis of Sera for Anti-Peptide IgG Antibodies

Each serum sample was analyzed by enzyme-linked immunoadsorbent assay (ELISA) Polystyrene microtiter plates were coated with 0.5 μg per well of the synthetic peptide (not conjugated to Omp) in phosphate-buffered physiological saline (PBS) at 4°C. Each well was then washed with PBS containing 0.05% TWEEN-20 (PBS-T). Test serum, diluted serially in PBS-T, was added to the peptide-containing wells and allowed to react with the adsorbed peptide for one hour at 36°C. After washing with PBS-T, alkaline phosphatase-conjugated goat anti-human IgG was added to the test wells and was allowed to react for one hour at 36°C. The wells were then washed extensively in PBS-T. Each well received 0.1% p-nitrophenyl phosphate in 10% diethanolamine, pH 9.8, containing 0.5 mM $MgCl_2$ $6H_2O$. The ensuing reaction was allowed to proceed at room temperature for 30 minutes, at which time it was terminated by the addition of 3.0 N NaOH.

The greater the interaction of antibodies in the test serum with the peptide substrate, the greater is the amount of alkaline phosphatase bound onto the well. The phosphatase enzyme mediates the breakdown of p-nitrophenyl phosphate into a molecular substance which absorbs light at a wavelength of 405 nm. Hence, there exists a direct relationship between the absorbance at 405 nm of light at the end of the ELISA reaction and the amount of peptide-bound antibody.

All the monkeys inoculated with the peptide-Omp (RP135-Omp) conjugate developed antibodies specifically capable of binding the peptide, as indicated by the anti-RP135 Titer of Table A.

Table A

| Initial Evaluation of Immunogenicity[*] | | | | | |
|---|---|---|---|---|---|
| | | Anti-RP135 Titer[2] | | | |
| Animal # | Inoculum | wk 0 | wk 2 | wk 4 | wk 6 |
| 182 | RP135-Omp | <20 | <20 | 500 | 2500[3] |
| 161 | (Alum) | <20 | <20 | 100 | 2500[3] |
| 162 | | <20 | 100[3] | <20 | 2500[3] |
| 163 | | <20 | <20 | 20 | 500[3] |
| 171 | RP | <20 | <20 | <20 | <20 |
| 035 | Loop[4] | <20 | <20 | <20 | <20 |
| 036 | peptide | <20 | <20 | <20 | <20 |
| 150 | (Alum) | <20 | <20 | <20 | <20 |

[*]At weeks 0 and 4 (as well as weeks 8 and 28), each animal was bled, then injected intramuscularly with the RP135-Omp conjugate immunogen according to example 4.

[2]Reciprocal of end-point ELISA titer using RP135 peptide as substrate coating on microtiter plates.

[3]Reciprocal of end-point virus neutralization titer on these days = 10 or 20.

[4]Sequence (of control RP loop peptide) = CTRPNNNTRKSIRIQRGPGRAFVTIGKIGNMRQAHC.

18

## EXAMPLE 6

Analysis of Sera for Activity which Specifically Neutralizes HIV Infectivity

Virus-neutralizing activity was determined with an assay described by Robertson et al., J. Virol. Methods 20: 195-202 (1988). The assay measures specific HIV-neutralizing activity in test serum. The assay is based on the observation that MT-4 cells, a human T-lymphoid cell line, are readily susceptible to infection with HIV and, after a period of virus replication, are killed as a result of the infection.

The test serum was treated at 56° C for 60 minutes prior to the assay. This treatment is required to eliminate non-specific inhibitors of HIV replication. Heat treated serum, serially diluted in RPm-1640 cell culture medium, was mixed with a standard infection dose of HIV. The dose had been determined prior to the assay as containing the smallest quantity of virus required to kill all the MT-4 cells in the assay culture after a period of 7 days. The serum-virus mixture was allowed to interact for one hour at 37° C. It then was added to 1.0 x 10⁵ MT-4 cells suspended in RPMI-1640 growth medium supplemented with 10% fetal bovine serum. The cultures were incubated at 37° C in a 5% $CO_2$ atmosphere for 7 days.

At the end of the incubation period, a metabolic dye, DTT, was added to each culture. This dye is yellow in color upon visual inspection. In the presence of live cells, the dye is metabolically processed to a molecular species which yields a blue visual color. Neutralized HIV cannot replicate in the target MT-4 cells and therefore does not kill the cells. Hence, positive neutralization is assessed by the development of blue color following addition of the metabolic dye.

All the monkeys inoculated with the RP135-Omp conjugate developed specific HIV infectivity-neutralizing activity, as indicated by the asterisked bleedings of Table A.

Further follow-up evaluation of the same monkeys (182, 161, 162, and 163) demonstrated substantial HIV-neutralizing activity, as provided in Table B. Booster shots also elicited renewed neutralizing titer.

### Table B

Follow-Up Evaluation of RP135-Omp-Inoculated Animals

All the monkeys inoculated with the RP135-Omp conjugate developed specific HIV infectivity-neutralizing activity, as indicated by the bleedings noted in Table A.

Further follow-up evaluation of the same monkeys (182, 161, 162, and 163) demonstrated substantial HIV-neutralizing activity, as provided in Table B.

## TABLE B

### Follow-up Evaluation of RP135-Omp-Inoculated Animals

| Animal # | Week, post-inoculation[1] | Anti-RP135 ELISA Titer[2] | Neutralizing Activity[3] |
|---|---|---|---|
| 182 | 0 | <20 | <10 |
| | 2 | <20 | <10 |
| | 4 | 500 | <10 |
| | 6 | 2500 | 10 |
| | 8 | 2500 | 20 |
| | 10 | 2500 | 320 |
| | 12 | 500 | 80 |
| | 16 | 100 | 80 |
| | 20 | 20 | 10 |
| | 24 | 20 | <10 |
| | 28 | 20 | <10 |
| | 30 | 2500 | ≥320 |
| 161 | 0 | <20 | <10 |
| | 2 | <20 | <10 |
| | 4 | 100 | <10 |
| | 6 | 2500 | 20 |
| | 8 | 2500 | 10 |
| | 10 | 2500 | 40 |
| | 12 | 500 | 10 |
| | 16 | 100 | <10 |
| | 20 | 20 | <10 |
| | 24 | 20 | <10 |
| | 28 | 20 | <10 |
| | 30 | 2500 | 80 |
| 162 | 0 | <20 | <10 |
| | 2 | 100 | 10 |

| Animal # | Week, post-inoculation[1] | Anti-RP135 ELISA Titer[2] | Neutralizing Activity[3] |
|---|---|---|---|
| | 4 | <20 | <10 |
| | 6 | 2500 | 20 |
| | 8 | 500 | 10 |
| | 10 | 2500 | 320 |
| | 12 | 500 | 40 |
| | 16 | 100 | 40 |
| | 20 | 20 | <10 |
| | 24 | 20 | <10 |
| | 28 | 20 | <10 |
| | 30 | 2500 | 320 |
| 163 | 0 | <20 | <10 |
| | 2 | <20 | <10 |
| | 4 | 20 | <10 |
| | 6 | 500 | 20 |
| | 8 | 500 | 10 |
| | 10 | 500 | 80 |
| | 12 | 100 | 160 |
| | 16 | <20 | 40 |
| | 20 | 100 | <10 |
| | 24 | 20 | <10 |
| | 28 | 100 | <10 |
| | 30 | 2500 | 80 |

[1] Each animal was inoculated with 100 mg of the conjugate, alum-adsorbed, per dose. Doses were delivered intramuscularly at 0, 4, 8 and 28 weeks.

[2] Reciprocal of end-point ELISA titer using the RP135 peptide as substrate coating on microtiter plates.

[3] Reciprocal of end-point virus-neutralization titer.

While the foregoing specification teaches the principles of the present invention, with examples provided for the purpose of illustration, it will be understood that the practice of the invention encompasses all of the usual variations, adaptations, or modifications, as come within the scope of the following claims and its equivalents.

**Claims**

1. An antigenic conjugate of HIV major neutralization determinant covalently linked to purified outer membrane proteosome of Neisseria.

2. The antigenic conjugate of claim 1 wherein said conjugate is of the formula

$(MNtD)_n \sim\!\!\sim (Omp)$

wherein:

MNtD is the major neutralization determinant of HIV, which is a polypeptide of one or more amino acid sequences;

n indicates the number of polypeptides of MNtD covalently linked to Omp and is 1-50;

$\sim\!\!\sim$ indicates covalent linkage;

Omp is purified outer membrane proteosome of Neisseria,

or pharmaceutically acceptable salt thereof, said MNtD being immunologically cross-reactive with one or more of the following peptides;

YNKRKRIHIGPGRAFYTTKNIIGTI,

NNTTRSIHIGPGRAFYATGDIIGDI, or

NNTRKSIRIQRGPGRAFVTIGKIGN.

3. The antigenic conjugate of claim 1 wherein said conjugate is of the formula

$(MNtD)_n \sim\!\!\sim (Omp)$

wherein:

MNtD is the major neutralization determinant of HIV, which is a polypeptide of one or more amino acid sequences;

n indicates the number of polypeptides of MNtD covalently linked to Omp and is 1-50;

$\sim\!\!\sim$ indicates a covalent linkage;

Omp is purified outer membrane proteosome of Neisseria,

or pharmaceutically acceptable salt thereof, said MNtD being a polypeptide having a sequence of 44 amino acids or less, but at least 5 amino acids in length, of the formula;

$C\text{-}J_1\text{-}G\text{-}X\text{-}G\text{-}J_2\text{-}C$

wherein:

C is absent or cysteine;

$J_1$ is absent or any peptide up to 19 amino acids in length;

G is glycine;

X is proline, leucine, alanine, glutamine or serine; and

$J_2$ is absent or any peptide of up to 20 amino acids in length.

4. The antigenic conjugate of claim 3 wherein X is proline.

5. The antigenic conjugate of claim 3 wherein X is proline, C is present twice and both $J_1$ and $J_2$ are present.

6. The antigenic conjugate of claim 3 wherein MNtD is further defined as any of the isolated equivalents of MNtD, fragment or variant thereof, said MNtD, fragment or variant thereof being 5 or more amino acids in length and immunologically cross-reactive with one or more of the following peptides:

YNKRKRIHIGPGRAFYTTKNIIGTI,

NNTTRSIHIGPGRAFYATGDIIGDI, or

NNTRKSIRIQRGPGRAFVTIGKIGN.

7. The antigenic conjugate of claim 3 wherein MNtD is further defined as any of the isolated equivalents of MNtD of Table II.

8. The antigenic conjugate of claim 2 wherein the conjugate is a covalent conjugate of Omp of Neisseria and the determinant having the peptide sequence of YNKRKRIHIGPGRAFYTTKNIIGTI (of the MN isolate).

9. The antigenic conjugate of claim 2 wherein the conjugate is a covalent conjugate of Omp of Neisseria and the determinant having the peptide sequence of NNTTRSIHIGPGRAFYATGDIIGDI (of the SC isolate).

10. The antigenic conjugate of claim 2 wherein the conjugate is a covalent conjugate of Omp of Neisseria and the determinant having the peptide sequence of NNTRKSIRIQRGPGRAFVTIGKIGN (of the IIIB isolate).

11. The antigenic conjugate of claim 2 wherein the conjugate is a covalent conjugate of Omp of Neisseria and the determinant having the consensus peptide sequence LNESVEINCTRPNNNTRKSIHIGPGRAFYTTGRIIGDIRQAHC.

12. The antigenic conjugate of claim 2 wherein the conjugate is a covalent conjugate of Omp of Neisseria and a determinant of 5 or more amino acids with the consensus peptide sequence.

13. The antigenic conjugate of claim 2 wherein the covalent linkage between MNtD and Omp consists essentially of a bigeneric spacer.

14. The antigenic conjugate of claim 3 wherein the covalent linkage between MNtD and Omp consists essentially of a bigeneric spacer.

15. The antigenic conjugate of claims 1-14, in combination with any of the antivirals, immunomodulators, antibiotics or vaccines of Table III.

16. The antigenic conjugate of claims 1-3, 8-14 wherein said Omp is derived from Neisseria meningitidis.

17. The antigenic conjugate of' claim 1, consisting essentially of $(RP135)_{3.3}$-Omp having a covalent linkage consisting of a bigeneric spacer, said Omp being purified from Neisseria meningitidis.

18. A cocktail of antigenic conjugates consisting essentially of a mixture of more than one molecular species of the antigenic conjugates of claims 1-14, or 17.

19. An AIDS vaccine comprising an antigenic conjugate of HIV major neutralization determinant covalently linked to purified outer membrane proteosome of Neisseria, said conjugate mixed with a suitable immunological adjuvant, carrier or vector, said vaccine to be used pre- and post-exposure to prevent or treat HIV infection or disease, said vaccine capable of eliciting specific HIV neutralizing antibodies.

20. The AIDS vaccine of claim 19, wherein the antigenic conjugate is of the formula
$(MNtD)_n \sim (Omp)$
wherein
MNtD is the major neutralization determinant of HIV, which is a polypeptide of one or more amino acid sequences;
n indicates the number of polypeptides of MNtD covalently linHed to Omp and is 1-50:
$\sim$ indicates covalent linkage;
Omp is purified outer membrane proteosome of Neisseria,
or pharmaceutically acceptable salt thereof, said MNtD being immunologically cross-reactive with one or more of the following peptides:
YNKRKRIHIGPGRAFYTTKNIIGTI,
NNTTRSIHIGPGRAFYATGDIIGDI, or
NNTRKSIRIQRGPGRAFVTIGKIGN.

21. The AIDS vaccine of claim 19,, wherein the antigenic conjugate is of the formula
$(MNtD)_n \sim (Omp)$
wherein
MNtD is the major neutralization determinant of HIV, which is a polypeptide of one or more amino acid sequences:
n indicates the number of polypeptides of MNtD covalently linked to Omp and is 1-50;
$\sim$ indicates a covalent linkage;
Omp is purified outer membrane proteosome of Neisseria,
or pharmaceutically acceptable salt thereof,
said MNtD being a polypeptide having a sequence of 44 amino acids or less, but at least 5 amino acids in length, of the formula;
$C-J_1-G-X-G-J_2-C$
wherein:
C is absent or cysteine:
$J_1$ is absent or any peptide up to 19 amino acids in length:
G is glycine;
R is proline, leucine, alanine, glutamine or serine; and
$J_2$ is absent or any peptide of up to 20 amino acids in length.

22. The AIDS vaccine of claim 21, wherein X is proline.

23. The AIDS vaccine of claim 21, wherein X is proline, C is present twice and both $J_1$ and $J_2$ are present.

24. The AIDS vaccine of claim 21, wherein MNtD is further defined as any of the isolated equivalents of MNtD, fragment or variant thereof, said MNtD, fragment or variant thereof being 5 or more amino acids in length and immunologically cross-reactive with one or more of the following peptides
YNKRKRIHIGPGRAFYTTKNIIGTI.
NNTTRSIHIGPGRAFYATGDIIGDI. or
NNTRKSIRIQRGPGRAFVTIGKIGN.

25. The AIDS vaccine of claim 21, wherein MNtD is further defined as any of the isolated equivalents of MNtD of Table II.

26. The AIDS vaccine of claim 20 wherein the antigenic conjugate is a covalent conjugate of Omp of Neisseria and the determinant having the peptide sequence of YNKRKRIHIGPGRAFYTTKNIIGTI (of the MN isolate).

27. The AIDS vaccine of claim 20 wherein the antigenic conjugate is a covalent conjugate of Omp of Neisseria and the determinant having the peptide sequence of NNTTRSIHIGPGRAFYATGDIIGDI (of the SC isolate).

28. The AIDS vaccine of claim 20 herein the antigenic conjugate is a covalent conjugate of Omp of Neisseria and the determinant having the peptide sequence of NNTRKSIRIQRGPGRAFVTIGKIGN (of the IIIB isolate).

29. The AIDS vaccine of claim 20 wherein the antigenic conjugate is a covalent conjugate of Omp of Neisseria and the determinant having the consensus peptide sequence LNESVEINCTRPNNNTRKSIHIGPGRAFYTTGRIIGDIRQAHC.

30. The AIDS vaccine of claim 20 wherein the antigenic conjugate is a covalent conjugate of Omp of Neisseria and a determinant of 5 or more amino acids with the consensus peptide sequence.

31. The AIDS vaccine of claim 20 wherein the covalent linkage between MNtD and Omp consists essentially of a bigeneric spacer.

32. The AIDS vaccine of claim 21 wherein the covalent linkage between MNtD and Omp consists essentially of a bigeneric spacer.

33. The AIDS vaccine of claims 19-32, in combination with any of the antivirals, immunomodulators, antibiotics or vaccines of Table III.

34. The AIDS vaccine of claims 19-21, 26-32 wherein said Omp is derived from Neisseria meningitidis.

35. The AIDS vaccine of claim 19, wherein the antigenic conjugate consists essentially of (RP135)$_{3.3}$-Omp having a covalent linkage consisting of a bigeneric spacer, said Omp being purified from Neisseria meningitidis.

36. The AIDS vaccine of claims 19-21, 26-30 or 35, comprising a cocktail of said antigenic conjugates, said cocktail consisting essentially of a mixture of more than one molecular species of said antigenic conjugates.

37. A pharmaceutical composition comprising an antigenic conjugate of HIV major neutralization determinant covalently linked to purified outer membrane proteosome of Neisseria. said conjugate mixed with a suitable immunological adjuvant, said composition useful as a vaccine capable of producing specific HIV neutralizing antibody in mammals.

38. The use of an antigenic conjugate of HIV major neutralization determinant covalently linked to purified outer membrane proteosome of Neisseria, said conjugate mixed with a suitable immunological adjuvant, for the preparation of a composition useful for vaccinating against AIDS.

24